**Europäisches Patentamt**

**European Patent Office**

**Office européen des brevets**

(11) Veröffentlichungsnummer: **0 038 507**
**A1**

# (12) EUROPÄISCHE PATENTANMELDUNG

(21) Anmeldenummer: **81102834.9**

(22) Anmeldetag: **14.04.81**

(51) Int. Cl.³: **C 07 C 120/00**
**C 07 C 121/16**

(30) Priorität: **17.04.80 DE 3014729**

(43) Veröffentlichungstag der Anmeldung:
**28.10.81 Patentblatt 81/43**

(84) Benannte Vertragsstaaten:
**BE DE FR GB IT NL**

(71) Anmelder: **Ruhrchemie Aktiengesellschaft**
**Bruchstrasse 219**
**D-4200 Oberhausen 13(DE)**

(72) Erfinder: **Horn, Gerhardt, Dr. Dipl.-Chem.**
**Bunsenstrasse 19**
**D-4200 Oberhausen 13(DE)**

(72) Erfinder: **Frohning, Dieter, Dr. Dipl.-Chem.**
**Elsenbruch 1**
**D-4200 Oberhausen 13(DE)**

(72) Erfinder: **Liebern, Hans**
**Auf dem Dudel 15**
**D-4330 Mülheim/Ruhr(DE)**

(74) Vertreter: **Reichelt, Karl-Heinz, Dr.**
**m. Br. Ruhrchemie Aktiengesellschaft Abt. PLD Postfach**
**13 01 60**
**D-4200 Oberhausen 13(DE)**

(54) Verbessertes Verfahren zur Herstellung von Carbonsäurenitrilen.

(57) Zur Herstellung von Nitrilen setzt man Alkohole oder Aldehyde mit Ammoniak in Gegenwart von Kupferkatalysatoren um. Die Aktivität der Katalysatoren wird erfindungsgemäß dadurch verbessert, daß man die Reaktion in Gegenwart von 20 bis 50 Vol.-% Wasserstoff (bezogen auf das Ammoniak-/Wasserstoffgemisch) durchführt.

EP 0 038 507 A1

COMPLETE DOCUMENT

Croydon Printing Company Ltd.

0038507

Oberhausen 13, 11.04.1980
PLD rcht-evr   R 1887

Ruhrchemie Aktiengesellschaft, Oberhausen 13

## Verbessertes Verfahren zur Herstellung von Carbonsäurenitrilen

Die Erfindung betrifft ein verbessertes Verfahren zur Herstellung von Nitrilen durch katalytische Umsetzung von Alkoholen mit Ammoniak. Durch Zusatz von Wasserstoff zum Ammoniak wird die Lebensdauer des Katalysators wesentlich verlängert und es werden höhere Produktausbeuten erzielt.

Zur Herstellung von Carbonsäurenitrilen sind bereits verschiedene Verfahren bekannt.

Ein klassisches Darstellungsverfahren für Nitrile stellt die Dehydratisierung der Ammoniumsalze von Carbonsäuren beziehungsweise die Umsetzung von Carbonsäuren mit Ammoniak unter Wasserabspaltung an dehydratisierenden Katalysatoren wie Silicagel oder $Al_2O_3$ bei Temperaturen von 360 bis 500°C dar (A.C. Cope, R.J. Cotter u. L.L. Esters, Org. Syntheses 34,4 [1954]).

Ein Nachteil dieser Herstellungsmethode ist die erforderliche hohe Reaktionstemperatur und der damit verbundene hohe Energiebedarf. Entsprechendes gilt auch für die katalytische Dehydratisierung von Amiden.

- 2 -

z. B. an $Al_2O_3$ (D. Boehmer u. F. Andrews, J. Amer. Chem. Soc. 38, 2504[ 1916]).Die Überführung von Säureamiden auf nichtkatalytischem Wege mit Hilfe von Dehydratisierungsmitteln wie Aluminiumchlorid, Phosphorpentoxid, Phosphorchloriden, Thionylchlorid u.a. zu den entsprechenden Nitrilen ist zwar für die meisten Nitrile anwendbar, jedoch nur in wenigen Fällen wirtschaftlich.

Vorteilhafte Ausgangsstoffe für die Herstellung von Nitrilen sind in vielen Fällen Aldehyde oder Alkohole.

Insbesondere wenn die entsprechenden Aldehyde bzw. Alkohole durch großtechnische Verfahren, z. B. Oxosynthese erhalten werden, ist es vorteilhaft, von diesen Verbindungen als Einsatzstoffen auszugehen.

So kann die Herstellung von i-Butyronitril durch Umsetzung von i-Butyraldehyd mit Ammoniak an $ThO_2$-Katalysatoren (F. Mailhe, Compt. Rend. 166, 216,1918) an Silber (US-PS 23 37 421) oder Kupfer (US-PS 23 37 422) auf dehydratisierenden Trägermaterialien erfolgen.

Entsprechend werden auch andere Alkohole wie n-Butanol mit $NH_3$ an Molybdän (VI)-oxid/$Al_2O_3$ (US-PS 24 87 299) bzw. an Nickel/$Al_2O_3$ (*C.A. 52, 19925 [1958])und Propanol in die entsprechenden Nitrile überführt.

*M.A. Popow u. N.J. Shufkin, Izvest. Akad. Nouk SSSR, Otdel.Khun.Nouk (1958);

Bei der Umsetzung von Aldehyden oder Alkoholen mit
Ammoniak in der Gasphase an den genannten Katalysatoren tritt bereits nach relativ kurzer Betriebszeit ein Nachlassen der Katalysator-Aktivität und
gleichzeitig damit die Bildung unerwünschter Nebenprodukte auf. Durch übliche Maßnahmen wie Temperaturerhöhung und Änderung der Verweilzeit und der Katalysatorbelastung kann nur kurzfristig eine Verbesserung des Katalysatorverhaltens erreicht werden.
Sie wird jedoch in den meisten Fällen mit einer
raschen Desaktivierung erkauft.

Es bestand daher die Aufgabe, ein Verfahren zu entwickeln, das es gestattet, Alkohole oder Aldehyde
in Gegenwart von Katalysatoren unter Vermeidung der
geschilderten Nachteile in Nitrile zu überführen.

Erfindungsgemäß erfolgt die Herstellung von Nitrilen
durch Umsetzung von Alkoholen oder Aldehyden mit
Ammoniak in Gegenwart von Kupfer-Trägerkatalysatoren.
Die neue Arbeitsweise ist dadurch gekennzeichnet,
daß die Reaktion in Gegenwart von 20 bis 50 Vol.-%
Wasserstoff, bezogen auf das Ammoniak-Wasserstoffgemisch, erfolgt.

Überraschenderweise wird das Langzeitverhalten von
Kupfer-Trägerkatalysatoren durch die erfindungsgemäße Maßnahme entscheidend verbessert. Dieser
Befund ist unerwartet, da sowohl bei der Umsetzung
von Alkoholen als auch von Aldehyden mit Ammoniak bei den angewandten Temperaturen von 270 bis 320°C Wasserstoff freigesetzt wird und damit bei Zusatz von Wasserstoff eher mit einer Umsatz- oder Selektivitätsminderung zu rechnen ist. Tatsächlich bewirkt jedoch der Zusatz von Wasserstoff eine Verbesserung der Selektivität hinsichtlich der Nitrilbildung und insbesondere eine wesentliche Verlängerung der Lebensdauer des Katalysators bei konstant hohem Umsatz.

Während nach den Verfahren des Standes der Technik die Katalysator-Betriebszeit bis zur ersten Regenerierung maximal 3 bis 4 Wochen beträgt, ist bei Anwendung der neuen Arbeitsweise auch nach 3-monatiger Betriebszeit kein Nachlassen der Katalysatorleistung festzustellen.

Durch Zusatz von Wasserstoff wird überdies der Alkohol- bzw. Aldehyd-Umsatz gegenüber Verfahren, die ohne Wasserstoffzugabe arbeiten, von max. 90 bis 95 % auf 99,0 bis 99,5 % erhöht. Weiterhin vorteilhaft ist für das erfindungsgemäße Verfahren, daß die spezifische Beaufschlagung bzw. Katalysatorbelastung von früher 0,2 V/Vh auf 0,28 V/Vh bei der neuen Fahrweise mit $H_2$ gesteigert werden kann.

Zusammengefaßt ermöglicht das neue Verfahren eine vorteilhaftere Herstellung von Nitrilen aus den entsprechenden Alkoholen oder Aldehyden, wobei insbesondere durch die erhebliche Verbesserung des Katalysator-Langzeitverhaltens die Wirtschaftlichkeit des Verfahrens entscheidend verbessert werden konnte.

Der Wasserstoff wird zusammen mit dem Ammoniak und dem Alkohol bzw. Aldehyd über den Katalysator geleitet. Die Konzentration des Wasserstoffes im Gasgemisch beträgt 20 bis 50 Vol.-%, insbesondere 25 bis 35 Vol.-%.

Als Alkohole können nach der neuen Arbeitsweise z. B. n- oder i-Propanol, 2-Ethylbutanol, 2-Ethyl-3-methylpentanol, 2-Ethylhexanol und insbesondere n- und/oder i-Butanol eingesetzt werden. Statt der Alkohole lassen sich mit gleich gutem Erfolg auch die Aldehyde gleicher Kohlenstoffatomzahl als Einsatzstoffe verwenden.

Alkohole bzw. Aldehyde und Ammoniak werden im Molverhältnis 0,1 : 1 bis 0,5 : 1 zur Reaktion gebracht, wobei Kupfer auf einem Trägermaterial als Katalysator dient. Im allgemeinen wendet man Katalysatoren an, die 10 bis 80 Gew.-% Kupfer, bezogen auf den gesamten reduzierten Katalysator, enthalten. Trägermaterialien sind z. B. $SiO_2$, z. B. in Form von Kieselgel oder Kieselgur, $Al_2O_3$, MgO sowie Aluminiumsilikate oder Gemische aus $Al_2O_3$ und $SiO_2$; bevorzugt werden Träger auf Basis $SiO_2$. Die Raumgeschwindigkeit bezogen auf flüssigen Alkohol oder Aldehyd beträgt 0,1 bis 0,3 $\frac{V}{V_{Kat.} \cdot h}$

Der günstige Einfluß von Wasserstoff bei der Umsetzung von Alkoholen oder Aldehyden mit Ammoniak in der Gasphase an Kupfer-Trägerkatalysatoren kann darauf zurückgeführt werden, daß Wasserstoff die Ausbildung aktivitätsmindernder Ablagerungen auf der Katalysatoroberfläche verhindert oder zumindest verzögert. Dadurch wird die Aktivität und Selektivität des Katalysators über einen wesentlich längeren Zeitraum auf einem durchschnittlich höheren Niveau gehalten als bei der bisher üblichen Arbeitsweise. Bei Anwendung des erfindungsgemäßen Verfahrens braucht man die Regenerierung des Katalysators erst nach verhältnismäßig langen Betriebszeiten durchzuführen, in günstigen Fällen kann man auf sie ganz verzichten.

Bei technischer Durchführung der neuen Arbeitsweise im kontinuierlichen Betrieb ist es lediglich beim Anfahren der Anlage nötig, die erforderliche Wasserstoffmenge zuzusetzen, da durch den Gaskreislauf der bei der Synthese gebildete Wasserstoff zwangsläufig zurückgeführt wird. Bei laufendem Betrieb muß dann nur dafür gesorgt werden, daß die Wasserstoffkonzentration im Kreisgas 20 bis 50 Vol.-% beträgt.

Sollte bei besonders langen Betriebszeiten ein Nachlassen der Katalysatoraktivität auftreten, so kann der Katalysator in der bekannten Weise durch eine stufenweise Behandlung mit Luft-Stickstoff-Gemischen im Temperaturbereich von 160 bis 320°C in situ regeneriert werden, unter allmählicher Steigerung des Sauerstoffgehaltes von 1 % auf 20 %.

Die anschließende Reduktion wird vorteilhaft mit einem Wasserstoff-Stickstoff-Gemisch (3 Vol.-% $H_2$) ebenfalls im Temperaturbereich von 160 bis 320°C und einer Raumgeschwindigkeit von

$$1000 \ \frac{V_{Gas}}{V_{Kat.} \cdot h} \ N_2 \ \text{durchgeführt.}$$

Beispiel 1

In einem Quarzrohr mit einem inneren Durchmesser von 20 bis 22 mm, das sich in einem elektrisch beheizten Aluminium-Blockofen befindet, werden über 150 ml eines reduzierten Kupferkatalysators (etwa 50 % Cu auf $SiO_2$) bei 290°C etwa 40 ml/h i-Butanol (0,27 V/Vh) gemeinsam mit 40 Nl/h $NH_3$ und 18 Nl/h $H_2$ geleitet. Das anfallende Reaktionsprodukt trennt sich in eine organische und in eine wäßrige Phase. Die organische Phase besteht zu 88 bis 90 % aus i-Butyronitril, 5 bis 6 % i-Butylamin, 1,5 bis 2 % Di-i-butylamin, 1 bis 2 % Schiff'scher Base und 0,5 bis 0,7 % nicht umgesetztem i-Butanol. Die wäßrige Phase, die etwa 25 % des gesamten Reaktionsproduktes beträgt, enthält etwa 5 bis 6 % i-Butyronitril neben 2 bis 3 % nicht identifizierten Komponenten und etwa 22 Gew.-% $NH_3$.

Über das Langzeitverhalten des Katalysators gibt Tab. 1 Auskunft.

Tab. 1: Herstellung von i-Butyronitril aus i-Butanol und $NH_3$ bei Normaldruck in Gegenwart von Wasserstoff

| Betriebstage | Reaktortemperatur °C | V/Vh | Einsatz $NH_3$ \| $H_2$ $Nm^3$ pro kg Einsatzprod. | | GC-Analysen; Durchschnittswerte | | | | | |
|---|---|---|---|---|---|---|---|---|---|---|
| | | | | | i-Butylamin | Schiffsche Base | Di-i-butylamin | i-Butyronitril | i-Butanol | Nachlauf |
| 1 - 10 | 290 | 0,26 | 1,3 - 1,5 | 0,6 | 6,1 | 1,0 | 1,5 | 89,5 | 0,6 | 1,3 |
| 11 - 28 | 290 | 0,27 | " | 0,6 | 5,9 | 1,2 | 2,0 | 90,0 | 0,5 | 0,4 |
| 29 - 50 | 290 | 0,27 | " | 0,6 | 6,5 | 1,5 | 2,5 | 88,0 | 0,5 | 1,0 |
| 51 - 65 | 290 | 0,27 | " | 0,6 | 7,9 | 1,2 | 2,0 | 87,7 | 0,7 | 0,5 |
| 66 - 74 | 300 | 0,27 | " | 0,6 | 5,0 | 1,2 | 1,0 | 91,8 | 0,4 | 0,6 |
| 75 - 77 | 300 | 0,27 | " | 0,6 | 5,8 | 1,5 | 2,6 | 86,9 | 0,6 | 0,6 |
| 78 - 82 | 310 | 0,27 | " | 0,6 | 6,2 | 1,5 | 1,0 | 90,1 | 0,6 | 0,6 |

R 1887

0038507

Vergleichsversuch 1:

Über einen reduzierten Kupferkatalysator werden
anfangs bei 280°C 30 ml/h bzw. 40 ml/h i-Butanol
gemeinsam mit 25 bis 30 Nl/h NH$_3$ jedoch ohne H$_2$-
Zusatz geleitet. Während der ersten 10 Betriebstage beträgt der Umsatz zu i-Butyronitril 90 bis
96 %, danach fällt er ab. Durch schrittweise Erhöhung der Reaktionstemperatur, zunächst auf 290°C
später auf 300°C, kann bis zum 26. Betriebstag
der Umsatz bei 85 bis 90 % i- Butyronitril gehalten
werden. Nach 30 Betriebstagen fällt die Leistung
des Katalysators so stark ab, daß der Versuch
abgebrochen werden muß.

Im Vergleich zu den Ergebnissen nach Beispiel 1
wird bei der Arbeitsweise ohne Zusatz von Wasserstoff eine wesentlich kürzere Laufzeit des Katalysators erreicht.

Solche in ihrer Leistung abgefallene Katalysatoren
lassen sich zwar in bekannter Weise mit Sauerstoff-
Stickstoff-Gemischen unter kontrollierten Bedingungen
regenerieren und erreichen nach Aktivierung durch
Reduktion nahezu wieder ihre ursprüngliche Leistungsfähigkeit. Nach der Regenerierung erfolgt der Abfall der Umsetzung zum gewünschten Nitril im allgemeinen rascher als in der ersten Fahrperiode.
Da durch die zwischenzeitliche Regenerierung die
auf die gesamte Betriebszeit bezogene Katalysatorleistung gemindert wird, ist das beanspruchte Verfahren, das wesentlich längere Katalysatorbetriebszeiten bei hohem Umsatz ermöglicht, der bisherigen
Verfahrensweise deutlich überlegen.

Beispiel 2:

Für die Herstellung von 2-Ethylbutyronitril werden über 150 ml eines tablettierten Kupferkatalysators, der in der gleichen Weise wie in Beispiel 1 reduziert wird, bei 280°C stündlich 40 ml 2-Ethylbutanal in verdampftem Zustand gemeinsam mit 45 Nl/h NH$_3$ und 15 1 Nl/h H$_2$ geleitet.

Das anfallende Reaktionsprodukt trennt sich in eine wäßrige und eine organische Phase. Bis zu einer Laufzeit von etwa 80 Tagen besteht die organische Phase zu 95 - 98 % aus 2-Ethylbutyronitril. Der Umsatz des eingesetzten 2-Ethylbutanals ist bis auf einen Rest von ca. 0,2 Gew.-% nahezu vollständig. Erst nach etwa 80 Tagen Betriebszeit setzt ein allmählicher Rückgang der Bildung von 2-Ethylbutyronitril ein, der jedoch durch Steigerung der Reaktionstemperatur auf 290°C bis 320°C behoben werden kann.

Vergleichsversuch 2:

Werden über 150 ml des gleichen Kupferkatalysators wie in Beispiel 2 bei 280°C 30 bis 40 ml/h 2-Ethylbutanal im verdampften Zustand gemeinsam mit 45 Nl/h NH$_3$, jedoch ohne H$_2$-Zusatz geleitet, so tritt bereits nach etwa 26 Tagen Betriebszeit ein deutlicher Abfall der Katalysatorleistung auf. Während anfangs das organische Reaktionsprodukt ebenfalls zu 95 - 98 % aus dem gewünschten 2-Ethylbutyronitril besteht, fällt der Nitrilanteil nach etwa 26 Betriebstagen beschleunigt ab. Durch Temperaturerhöhung auf 300 bis 320°C läßt sich zwar der Umsatz zu 2-Ethylbutyronitril bis zu etwa 45 Betriebstagen auf 85 bis 90 % halten, danach wird jedoch der Leistungsabfall des Katalysators so stark, daß das Reaktionsprodukt infolge seiner Zusammensetzung nicht mehr

0038507

mit wirtschaftlich vertretbarem Aufwand auf
reines 2-Ethylbutyronitril aufgearbeitet werden kann.

Beispiel 3:

Leitet man über 150 ml eines wie nach Beispiel 1
reduzierten Kupferkatalysators bei 280 - 300°C
30 ml/h 2-Ethyl-3-methylpentanol gemeinsam mit
25 Nl/h $NH_3$ und 7 Nl/h $H_2$, so erhält man ein
Rohprodukt, dessen organische Phase zu 99 % aus
2-Ethyl-3-methylvaleronitril besteht. Während
der gesamten Betriebszeit von über 60 Tagen wird
kein Nachlassen der Katalysatorleistung beobachtet.

Wie in Beispiel 1 und 2 kann auch bei der Umsetzung
des 2-Ethyl-3-methylpentanols zu dem entsprechenden
2-Ethyl-3-methylvaleronitril der günstige Einfluß
des $H_2$-Zusatzes für die Lebensdauer des Katalysators
festgestellt werden.

Oberhausen 13, 11.04.1980
PLD rcht-evr    R 1887

Ruhrchemie Aktiengesellschaft, Oberhausen 13

### Patentanspruch

Verfahren zur Herstellung von Nitrilen durch Umsetzung von Alkoholen oder Aldehyden mit Ammoniak in Gegenwart von Kupfer-Trägerkatalysatoren, dadurch gekennzeichnet, daß die Reaktion in Gegenwart von 20 bis 50 Vol.-% Wasserstoff, bezogen auf das Ammoniak/Wasserstoffgemisch, erfolgt.

# EUROPÄISCHER RECHERCHENBERICHT

**Europäisches Patentamt**

| | EINSCHLÄGIGE DOKUMENTE | | | KLASSIFIKATION DER ANMELDUNG (Int. Cl.³) |
|---|---|---|---|---|
| Kategorie | Kennzeichnung des Dokuments mit Angabe, soweit erforderlich, der maßgeblichen Teile | betrifft Anspruch | | |
| X | US - A - 2 795 600 (UNION CARBIDE) <br> * Spalte 2, Zeilen 47 bis 51; <br> Beispiel 2 * <br> -- | Anspruch | | C 07 C 120/00 <br> C 07 C 121/16 |
| A | US - A - 2 365 721 (SHARPLES) <br> * Beispiele 5,6 * <br> -- | | | |
| D,A | US - A - 2 337 422 (RÖHM & HAAS) <br> * Spalte 1, Zeilen 10 bis 20; Spalte 2, <br> Zeilen 19 bis 23 * <br> ---- | | | **RECHERCHIERTE SACHGEBIETE (Int. Cl.³)** <br><br> C 07 C 120/00 <br> C 07 C 121/16 |

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| Berlin | 09-07-1981 | BREW |

EPA form 1503.1 06.78